# EUROPEAN PATENT APPLICATION

(11) **EP 1 239 284 A1**
(43) Date of publication of application: **11.09.2002**
(21) Application number: 01302110.0
(22) Date of filing: 08.03.2001
(51) Int. Cl.: G01N 33/53, G01N 33/543, G01N 33/58, G01N 33/542

(54) **Non-separation assay method and system using opaque particles**

(71) Applicant: THE TECHNOLOGY PARTNERSHIP PUBLIC LIMITED COMPANY, Melbourn, Royston, Hertfordshire SG8 6EE (GB)
(72) Inventor: Cassells, John, Houghton, Huntingdon, Cambs PE17 2BQ (GB); Cope, Tristan John, Cambridgeshire CB2 5PR (GB)
(74) Representative: Hallybone, Huw George

(57) **Abstract**

A method for performing a non-separation assay for determining the level of binding of one component to another. A first component is provided incorporating a fluorescent probe dissolved or suspended in solution. A substantially opaque particle is provided onto or into which is incorporated binding sites for the first component and optionally incorporating a dye or fluorophore of different emission spectrum to the first component. The opaque particle is immersed in a solution or suspension of the first component, and the opaque particle to settle out of the solution, or be transported to a fixed position by an applied force. The solution and opaque particle are illustrated with a beam of light such that the opaque particle is in the foreground and attenuates and illuminating beam before it passes into the solution beyond. The intensity of received light (fluorescence) from the first component over an area of the sample with an imaging or scanning detector from the same side of the sample as the illuminating light is determined, and the position of the second component in the sample is determined by detecting attenuation of the received light from the sample and/or by detecting the presence of received light from a dye incorporated in the second component. An apparatus, as well as opaque particles for performing the method are also provided.

## Description

This invention relates to an assay method and system for performing the same. The ability to quantify or detect the binding of one biological molecule to another molecule, cell or part of a cell is important in both drug discovery and clinical diagnostics. This may be for example the binding of antibodies to antigens; hormones to receptors; ligands to cell surface receptors, enzymes to substrates; nucleic acids to other nucleic acids; nucleic acids to proteins and viruses to cell surfaces.

Many diseases are characterised by binding or transport processes. In drug discovery the aim is to identify a means of enhancing or blocking the process. In clinical diagnostics the aim is to detect abnormal function of these processes; the presence of abnormal nucleic acid material; the presence of or increase or decrease in the presence of a biological molecule; or to identify foreign bodies (such as viruses or bacteria) to diagnose a disease so that appropriate treatment may be given.

The present invention seeks to provide a rapid and simple assay to detect and quantify binding and transport processes important in drug discovery and clinical diagnostics.

For the purpose of the following discussion "receptor" shall mean any biological molecule, cell or structure that binds another molecule, cell or structure. Similarly "ligand" shall mean any organic or inorganic molecule that binds to the "receptor". The discussion and examples will focus on the assay of a labelled ligand binding to a receptor. The invention can be extended to include the interaction of a non-labelled agonist or antagonist in a competition assay as commonly used in drug discovery.

A well known assay used in drug discovery and diagnostics is the separation assay. In this assay one component (for example the ligand) is dissolved or suspended in solution. The other component (for example the receptor) may be immobilised to a surface such as the walls and/or base of a well in a microtitre plate, or may be present on the surface of a cell. One or both components may have a label such as a fluorescent or radioactive marker attached to it to assist measurement with an instrument. The assay is performed by adding the soluble component to a well containing the immobilised component and allowing the binding of the components to come to equilibrium. It is not possible with conventional detectors such as colorimetric, fluorescent or radioactivity plate readers to directly determine the amount of bound labelled ligand in the presence of free labelled ligand. This problem is overcome by separating the free ligand from the bound ligand by decanting off the solution containing the free ligand, leaving the bound ligand behind. This step is commonly combined with a wash step to assist in removing excess labelled ligand.

This assay technique has significant disadvantages. Firstly, if the rate of dissociation is fast some of the bound label will be released back into the wash solution resulting in an error during reading. The efficiency of washing itself may vary from one sample to the next, reducing the repeatability of the assay. It is desirable to reduce or eliminate washing steps in automated systems to increase throughput, reduce complexity and eliminate the risk of cross-contamination between samples.

A number of bead-based non-separation assay techniques have been developed in recent years to overcome these problems. Instead of coating one component onto the walls of the assay well, at least one component is attached to a bead. Various detection method are employed to measure the amount of an analyte bound to the assay beads without the need for wash steps. Common bead-based assay technologies include Scintillation Proximity Assay (SPA), Fluorescent Resonance Energy Transfer (FRET), and direct measurement of fluorescence on beads by the use of imaging or laser scanning systems or flow cytometry.

SPA relies on the transfer of energy from a radio labelled ligand to a scintillant bead onto which the receptor is attached. Legislation on the disposal of radioactive material and the risk of exposure to operators has led to companies seeking alternatives. Fluorescence-based assays are a viable alternative, providing a sensitivity comparable with SPA, but without the need for radioactive isotopes.

FRET is similar to SPA in that it relies on the transfer of energy from one molecule to another in close proximity. In this case energy from one fluorophore is transferred to another fluorophore in close proximity. Most FRET assay technologies require both the receptor and the ligand to be soluble and that a fluorophore be present on both the ligand and the receptor. This is not suitable for assays where a soluble receptor cannot be obtained, and in addition chemically modifying the receptor by the addition of a label can be difficult and lead to a reduction or elimination of activity. Amersham Pharmacia Biotech offer a FRET technology where the acceptor is present on a bead. This provides for a fluorescent homogeneous assay format similar in principle to SPA. However, the detection technique commonly employed is to measure the aggregate fluorescence of tens of thousands of beads in a well, and this does not facilitate material savings by allowing the assay to be miniaturised to a few tens or hundreds of beads.

Direct measurement of a labelled ligand binding to a bead or similar solid support may be measured by flow cytometry. This involves flowing the beads through a flow cell, and requires that a sample be pipetted out of the assay well for analysis. This involves an extra step in the process which is potentially slow. As a result, flow cytometry is not generally favoured for the large numbers of samples (up to 100,000 per day) found in screening for new drugs.

Direct measurement of a labelled ligand binding to a bead or similar solid support can also be performed by an imaging system or scanned laser. Observation or quantification of a fluorescently labelled ligand binding to a bead (or cell) is well known in the art. The advantages of using an imaging or scanned laser system are that no sampling step is required at detection, and the assay can be miniaturised to as little as a single bead. US5876946 describes the use of a confocal microscope to measure the brightness of beads or cells in a binding assay. It teaches the use of a pinhole to limit the amount of background signal from the solution (free fluorescence) . This approach is effective in allowing large depths of liquid to be used in an assay, but the use of a pinhole introduces a shallow depth of focus and thus makes the system intolerant of variations in the flatness of the substrate on which the beads are supported. Furthermore, the use of a pinhole cannot entirely eliminate the solution fluorescence so that in conditions where there is low binding of a labelled ligand to a bead it is not possible to detect the bead against the background free fluorescence.

PCT/GB00/00549 describes an alternative to the teaching of US5876946 in that it describes the use of a non-confocal scanned laser system to quantify the binding of a labelled ligand to a bead, cell or similar solid support without the use of a pinhole to limit the solution fluorescence. In this case, measurement of a significant signal from the free fluorescence is desirable because it can be used to compute the dissociation constant of the ligand or competing species. The non-confocal optics also facilitates the scanning of a larger area, which has benefits in providing higher information about the sample and higher speed for reading microplates. The method of PCT/GB00/00549 has the disadvantage that the depth of the liquid in the assay has to be kept within limits to avoid the signal from the free fluorescence becoming too strong. Alternatively, a quenching dye may be added to the solution to greatly reduce the background, but this cannot completely eliminate the free fluorescence.

The present invention provides a method and apparatus where the bound fluorescence on the bead can be measured with almost no interference from the free fluorescence while maintaining the ability to accurately measure the free fluorescence in any practical depth of liquid. The invention eliminates the need for confocal optics, thus facilitating wide area scanning of many wells at once and therefore better throughput. Furthermore, the method of the invention provides a means of detecting the assay beads even when little or no fluorescence is bound.

According to the present invention there is provided a method for performing a non-separation assay for determining the level of binding of one component to another, the method comprising the steps of:
providing a first component incorporating a fluorescent probe dissolved or suspended in solution;
providing a substantially opaque particle onto or into which is incorporated at least one of binding sites for the first component and a flourescent component;
immersing the opaque particle in a solution or suspension of the first component;
allowing the opaque particle to settle out of the solution, or be transported to a fixed position by an applied force;
illuminating the solution and opaque particle with a beam of light such that the opaque particle is in the foreground and attenuates the illuminating beam before it passes into the solution beyond;
determining the intensity of received light from the first component over an area of the sample with an imaging or scanning detector from the same side of the sample as the illuminating light; and
determining the position of the second component in the sample by detecting attenuation of the received light from the sample and/or the intensity of fluorescence from the second flourescent component.

The present invention may further comprise the step of determining the intensity of received light (fluorescence) from a dye incorporated in the second component.

The present invention also provides an apparatus for performing a non-separation assay, the apparatus comprising:
a opaque particle onto or into which is incorporated binding sites for a first component and/or a second flourescent component;
means for containing and presenting the first component and opaque particle in solution to an optical detector;
means for illuminating both the first component and the opaque particle with at least one illumination wavelength, the diameter of the beam or beams of light being no larger than twice the diameter of the opaque particle at the point of focus; and
means for measuring the intensity of the received light with at least one detection channel sensitive to the emission from the fluorophore incorporated in the first component.

The apparatus may have at least one additional detection channel sensitive to the emission from a fluorophore incorporated in the opaque particle and insensitive to the fluorophore incorporated in the first component.

The apparatus may further comprise means for performing a signal processing algorithm to pick out and isolate the intensity value of the first component bound to the opaque particle amongst the continuous background signal from the unbound first component by:
firstly determining the position of the opaque particle through the detection of an attenuation in the signal from at least one detection channel sensitive to the emission from the fluorophore incorporated in the first component and/or through the detection of an increase in the signal from at least one detection channel sensitive to the emission from the fluorophore incorporated in the opaque particle; and
secondly, measuring the intensity value from the detection channel sensitive to the emission from the fluorophore incorporated in the first component.

The first component may preferably be a fluorescently labelled protein, ligand, enzyme, enzyme substrate, nucleic acid sequence, small molecule, viral particle, bacterium, or biologically-coated microbead.

The opaque particle may be a polymeric bead containing an opaque filler or an opaque inorganic bead, such beads being coated with, made of or incorporating a protein, receptor, ligand, enzyme, substrate, cell membrane, nucleic acid sequence, plastic or other substance that will bind biological molecules and preferably being coated with or containing a fluorophore. The particle may be magnetic, paramagnetic, electrically charged or be of higher density than the sample solution to facilitate settling out of the particle by application of a magnetic field, electrophoresis, static charge, gravity or centrifugation.

The means of illumination is preferably a laser beam of no larger than twice the diameter of the first component and preferably of diameter equal to or smaller than the diameter of the opaque particle.

The prior art and the invention will now be described by reference to figures 1 to 10, in which:
Figure 1 is a schematic diagram of a known SA apparatus;
Figures 2A and 2B show outputs from the apparatus of figure 1;
Figure 3 is a schematic diagram of an apparatus according to the invention; and
Figures 4 to 10 show outputs of the apparatus of figure 3.

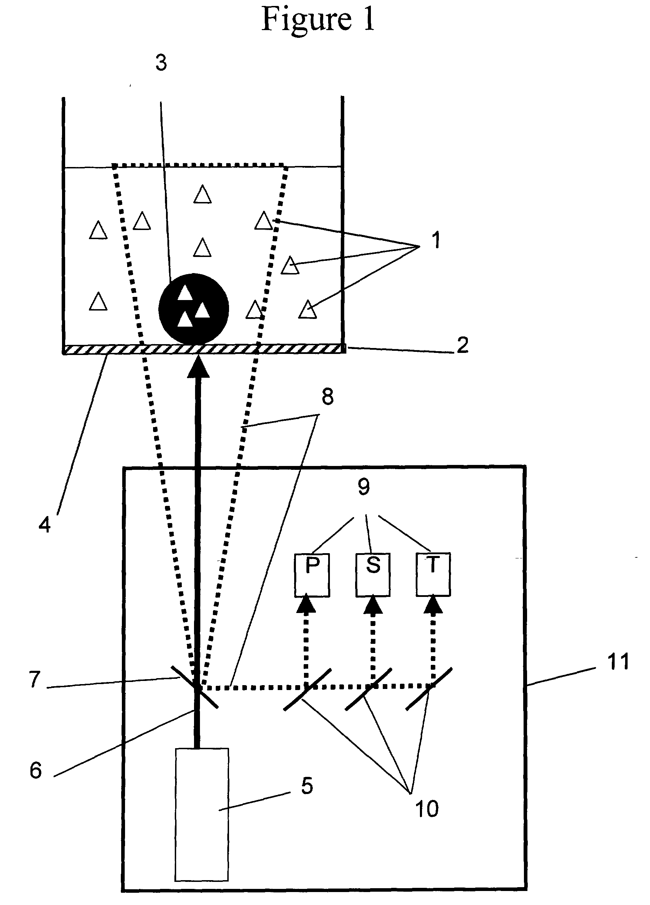

The prior art will now be described with reference to figure 1. In this a solution of a label 1 is contained in a receptacle 2. This receptacle 2 may be a microscope slide, well in a microtiter plate, flow cell in a flow cytometer or other suitable vessel. One or more opaque beads 3 are immersed in the label solution. These beads 3 have an affinity for the label 1 such that label 1 is absorbed and concentrated onto or into the beads 3 from the solution. There is provided an optical window 4, typically in the base of the receptacle, to allow observation of the beads 3. An imaging system 11 is provided. In this embodiment the imaging system 11 comprises a laser source 5 that emits a narrow beam of laser light 6. This laser light is scanned across the sample (scanning optics not shown) such that the beads 3 and label solution 1 are illuminated and the label 1 is excited to fluoresce. The portion of the resulting emitted light 8 that travels back along the path of the excitation may be separated from the laser light by a beam splitter 7 and quantified by a light sensitive detector 9. The emitted light may be further split into several emission bands by the use of optical filters 10 and extra detectors 9 to provide spectral information about the emissions from the beads 3 and label 1. Figure 1 shows the laser light paths 6 in one position only during a scan for illustration purposes. It will be appreciated that scanning is a continuous process. The laser beam 6 penetrates through the free solution where a bead 3 is not present, providing a measure of the concentration of label 1. Conventional polystyrene beads 1 used in the prior art for fluorescent assays are typically 2.6 to 20 microns in diameter. They are translucent such that the laser beam 6 passes through the bead 1 with little attenuation and illuminates the solution beyond. The emitted light received by the detector 10 is thus a combination of the emission of the label 1 bound to the bead 3 and of the free label 1 in solution behind the bead. In a non-confocal system, most of the light emitted by the free label 1 back towards the direction of illumination from the whole depth of the solution is detected by the detector. In a true confocal system light emitted outside of the confocal volume is substantially rejected.

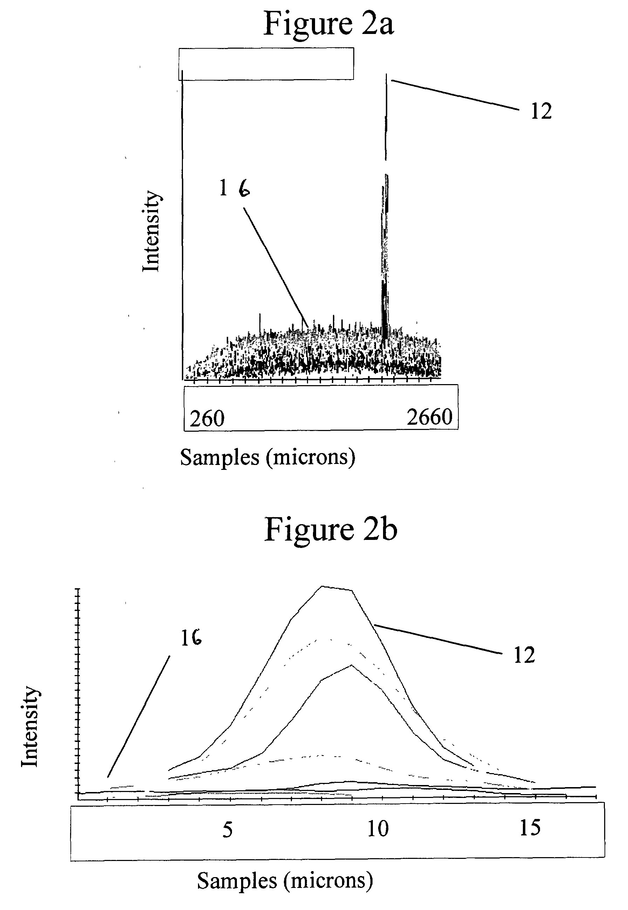

Figure 2a shows typical line amplitude signal obtained from scanning a bright bead in a solution of label with a laser scanning system (Acumen Explorer, The Technology Partnership, Cambridge, England). The continuous signal 16 represents the background signal from the free label. The peak 12 represents the signal from a bead that is much brighter than this background. Figure 2b shows typical line amplitude data for a bright bead in a shallow volume of liquid with the same instrument. Both confocal and non-confocal systems will give similar signal-to-background ratios at low liquid depths, but a confocal system will have superior signal-to-noise ratios at higher liquid depths. A threshold algorithm is used to determine the signal from the bead 3 above the background.

Performing assays by measuring the fluorescence of a substance binding to a bead relies on local concentration of a fluorescent label on the bead. If a bead is covered with a sufficient concentration of binding sites and the equilibrium constant (Kd) is favourable then the imaging system can resolve beads in the presence of free label because there will be a higher concentration of label on the bead than free in solution. The bead will therefore appear brighter than the surrounding free label, and this is the basis for all known prior art methods. The free label solution and the beads are commonly placed in a well in a clear-bottomed microtitre plate, and the beads allowed to settle. The bottom of the well is illuminated and imaged from below. In the method described by US5876946 a pinhole in the confocal system limits the background signal adjacent to the bead ensuring good contrast when the beads are coated with a significant amount of label. In drug screening the intention is to seek compounds that will displace the label from the beads. In this case, the bead intensity approaches that of the surrounding solution and cannot be detected.

The Applied Biosystems FMAT system operates the method of US5876946 and incorporates a pinhole to improve the contrast between the bead and free label. A reasonably reproduceable number of beads 3 are added to each assay well, and a commonly used assay approach is to count the number of beads detected as a measure of the potency of the test compound. The beads have a broad distribution of binding capacity for the label, and as the population of beads in the well become less bright, the less bright beads cannot be detected. This means that a dose-response curve will show a reducing number of beads detected.

The Acumen Explorer is a non-confocal system and operates the method of PCT/GB00/00549. This system does not have a pinhole to reject some of the signal from free label, and instead relies on higher resolution detection, and limiting of the liquid depth to ensure good contrast between the bead and background. As described in PCT/GB00/00549, assay beads are commonly translucent and thus the background signal is added to the signal on the bead helping to improve the contrast at deeper liquid volumes. The Acumen Explorer uses statistical analysis to accurately report the true mean and median of the population of beads, a method that is inherently more reproduceable than bead counting.

It is highly desirable for assays used both for clinical diagnostics and drug discovery that there are no false negatives or positives. The methods of both US5876946 and PCT/GB00/00549 have a serious disadvantage at the lower end of sensitivity in that neither method can confirm the number of beads present when some or all of the beads are of similar or less brightness to the background. Thus either system would give a result indicating the presence of a potent inhibitor of binding even if no beads had been added (a false positive). Further, pipetting beads is not highly reproducible and it is important to know precisely how many beads are in the assay. An excessive number of beads can cause depletion of the free label ( detected as a false positive). Having a precise count of the number of beads present also allows the system to normalise results from one well to the next.

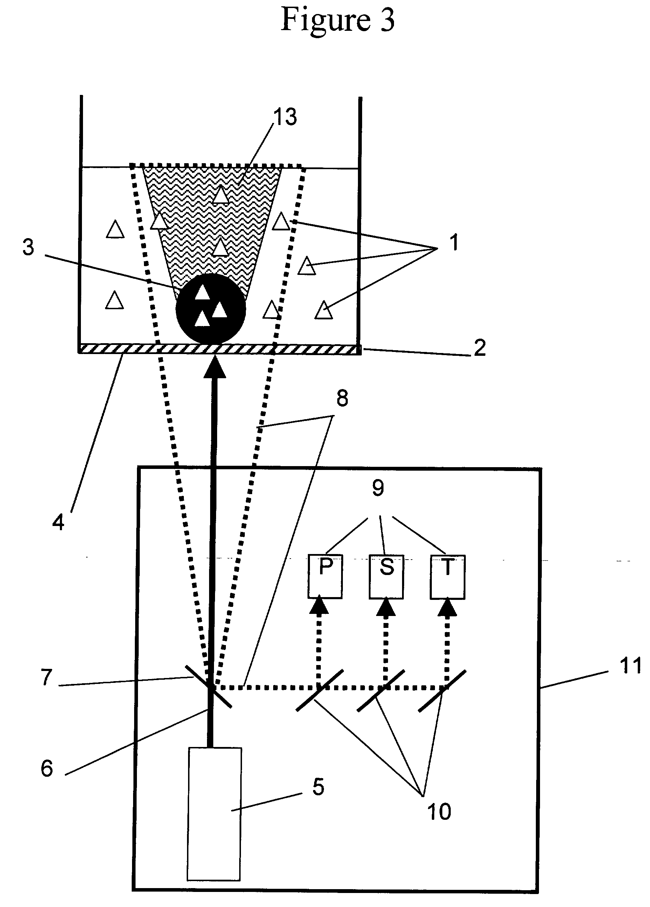

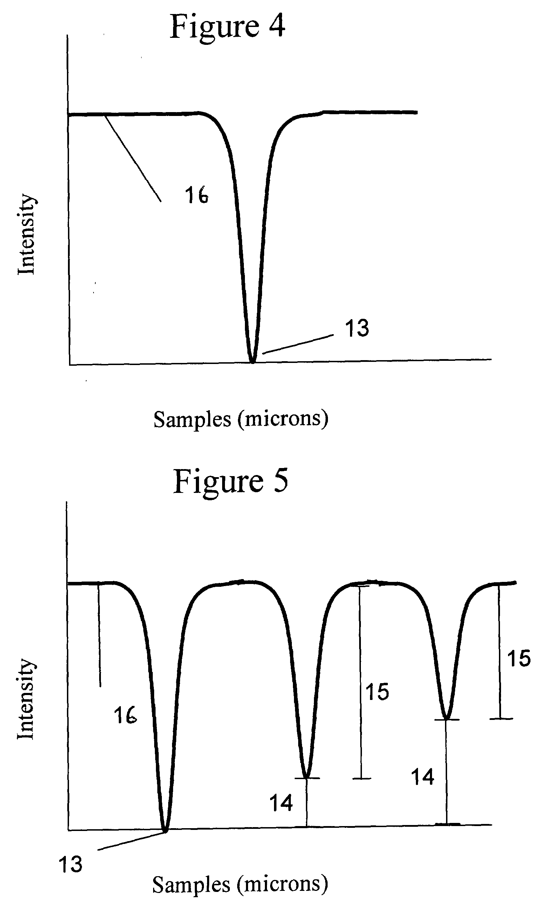

The present invention solves these problems. The invention is now described with reference to figure 3, using a laser scanning system 11 as an example of a suitable imaging/scanning system. It can be seen from figure 3 that an opaque bead 3 of a diameter comparable to or greater than the laser beam 6 casts a shadow 13 behind it when illuminated by a scanned laser. As the bead 3 is on the bottom of the well 4, a significant proportion (or all) of the solution above it is not illuminated when the beam is eclipsed and gives rise to no fluorescence signal. Only a negligible amount of free label can fill the space between the spherical bead 1 and the bottom of the well 4. With reference to figure 4, an opaque bead 3 to which no fluorophore-labelled ligand is bound thus gives rise to a null in the line amplitude signal ("negative peak") 13 against the background signal of the free label 16. This null signal is obtained at high inhibitor concentration where no appreciable amount of label 1 is binding to the bead 3, or where the beads used are reference beads formulated without receptor. This is true of both a confocal and non-confocal system, however the non-confocal system will generate much higher signals for the free label and thus a greater contrast between the bead 3 and background. A negative threshold can be used to detect the beads 3 relative to the background. The bead 3 may not completely eclipse the solution if it is only partially opaque or is smaller than the laser beam or is not completely in alignment with the laser beam. In these cases a null signal 13 is still observed but may not reach down to the x axis.

Figure 5 represents an illustration of the principle of the invention. The line amplitude signals obtained from three beads are shown where the amount of label binding to the bead increases from left to right. Increasing fluorescence from ligand bound on the bead moves the minima 13 further from the x-axis. A measure of the amount of ligand bound to each bead is given by the height 14 of this minima above the x-axis or alternatively the depth of the dip in the signal 15. This principle can be applied to a typical dose-response calibration. As the concentration of inhibitor is reduced, the binding of labelled ligand to the bead will increase causing the minima 13 of the bead signal to rise up from the baseline. For a given fixed free label concentration, this minima is proportional to the concentration of inhibitor added. Provided that the background signal remains higher than the signal from the bead, the number of beads can always be established even when no label is bound to them. The limit of detection is also substantially reduced because it is possible to quantify label on the bead when this is far less than the background. If the beads used are larger than the diameter of the laser beam the bead can completely eclipse the laser beam and the resulting signals at high inhibitor concentration can be as low as the noise floor of the detector, allowing maximum sensitivity. Smaller beads and/or semi-opaque beads that only allow a partial eclipse of the laser beam may still be employed, but compensation has to be made for the light spill around or through the bead.

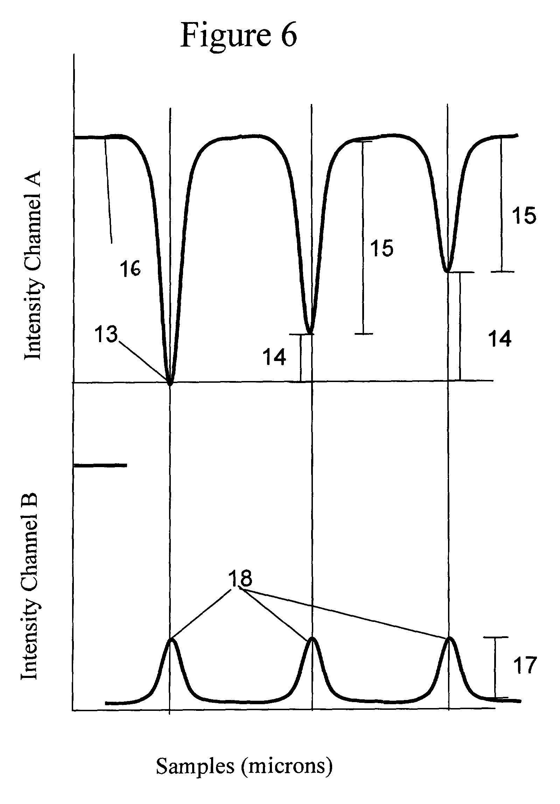

A further enhancement to the invention is to incorporate a dye into the beads 3 of a different emission spectra from the free label. This can be used to detect the beads 3 in a second channel when the intensity of the bead in the first (label) channel is close to that of the background. This is illustrated in figure 6. The top plot shows the line amplitudes for the channel that is tuned to the emission of the labelled ligand (channel A). The bottom plot illustrates the corresponding line amplitudes for the channel that is tuned to the emission of the fluorophore used to dye the beads 3 (channel B). The beads 3 are first located by detecting an increase in the intensity 18 of the line amplitudes in channel B through the use of a threshold algorithm. This increase in intensity is fixed and is characteristic of the concentration 17 of dye on the bead 3. The amount of ligand binding to the bead is measured by considering the intensity measurements 14 in channel A that correspond to a bead detected in channel B.

The combination of an opaque bead 3 with a dye is more desirable than the use of plain dyed beads alone because normal fluorescent-dyed beads are translucent and therefore a fluorescence intensity signal from the free label behind the beads is added to the fluorescence on the beads, interfering with the measurement of the amount of free label that has become bound to the bead 3. In addition, there is often spectral overlap between a dye used on a bead 3 and the free label. It is desirable to keep the dye concentration in the bead to a minimum to reduce interference or quenching. An opaque bead 3 provides additional contrast over plain dyed beads.

Another advantage of the invention is that it is reasonably tolerant of poor flatness in the bottom of the wells.

The method of the invention has the added benefit that it differentiates the beads 3 from normal organic contamination. In prior art assays, it is common to find bright fluorescent particles of contamination in the assay. These may come from fragments of organic material (for example from cells), contaminants from the label solution, or may be formed by precipitation of the label with other components in the assay. The prior art detection methods rely on detecting particles that are brighter than the background. Contaminating particles can wrongly identified as beads. However, as these contaminating particles are organic they are translucent to the laser beam and can easily be discriminated from opaque beads 3 by the method of the present invention.

The shape and size of the cell or bead 3 may be obtained from the line amplitude signals and allows the volume of the bead 3 or cell (or component in the laser beam) to be estimated.

Where a dye is incorporated in the beads 3 this may be used as an internal standard. With reference to figure 6, the intensity value 14 in channel A due to the binding of labelled ligand to the bead 3 may be compared with the intensity value 17 in channel B due to the fluorophore incorporated in the beads 3. The ratios of the signals in channel A and B provide a measure of the amount of binding of the labelled ligand to the beads 3 where the signal from channel B serves as an internal standard.

The system can be calibrated using beads 3 of known size and fluorophore loading, and solutions of known depth and fluorophore concentration. Provided that the depth of the well in which the assay is being performed or the concentration of the labelled ligand is known it is possible to directly measure free ligand concentrations and to predict important parameters such as K_{d}, Kᵢ, receptor concentration and IC₅₀ in a single assay.

A substantial benefit of the invention is the ability to measure the amount of labelled ligand bound to the bead 3 in any volume or concentration of free label.

The invention will now be explained further by reference to an example.

### EXAMPLE 1

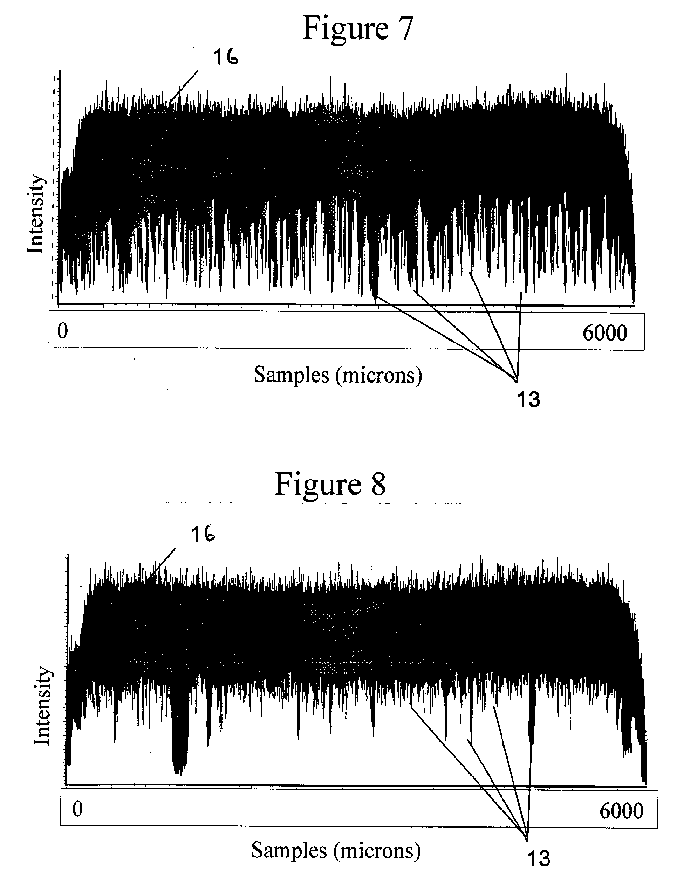

A volume of 50 ml of a solution of 5 nM of fluorescein isothiocyanate-labelled mouse polyclonal antibody (FITC-MOPC, Sigma Chemical Company, Poole, England) containing magnetite-loaded magnetic beads of 4.5 mm coated with streptavidin (Dynal, England) was pipetted into a well in a clear-bottomed 384-well microtitre plate. The resulting suspension was allowed to settle under gravity and was scanned by a non-confocal laser scanning system (Acumen Explorer, The Technology Partnership, Cambridge, England) having a laser beam diameter of 6 mm at the point of focus and a Gaussian energy profile across the beam. The line amplitudes obtained from one emission channel of the instrument are shown in figure 7. The fluorescent signal 11 due to the free fluorescently-labelled antibody can clearly be distinguished from the reduced signal 13 resulting from the attenuation of the laser beam by the substantially opaque magnetite-loaded beads. The beads did not completely eclipse the laser beam in this case as the beads were smaller than the diameter of the laser beam. However, as most of the energy of the laser beam was concentrated in an approximately 3 mm diameter centre of the beam, the beads gave sufficient attenuation to demonstrate the method of invention.

The experiment was repeated, substituting magnetite-loaded beads coated with sheep anti-mouse antibody (SAM beads, Dynal, England) for the streptavidin-coated beads (figure 8). The affinity of the SAM beads for the FITC-MOPC antibody resulted in a concentration of FITC-MOPC antibody on the beads and a corresponding increase in the fluorescence of the beads. Figure 8 shows a substantial reduction in the number of negative peaks attributable to non-fluorescent beads, and an increase in fluorescence of the beads that are detected. This is consistent with an increase in fluorescence on the beads due to FITC-MOPC antibody binding to the beads. Beads used in biological assays typically display a broad distribution of binding capacity. It was therefore expected that some beads would be detected that had not bound as much FITC-MOPC antibody as the rest of the population. An accurate assay can be performed by reporting the average brightness for the whole population of beads found in the well.

In this example a magnetic field could have been used to accelerate settling of the beads if required.

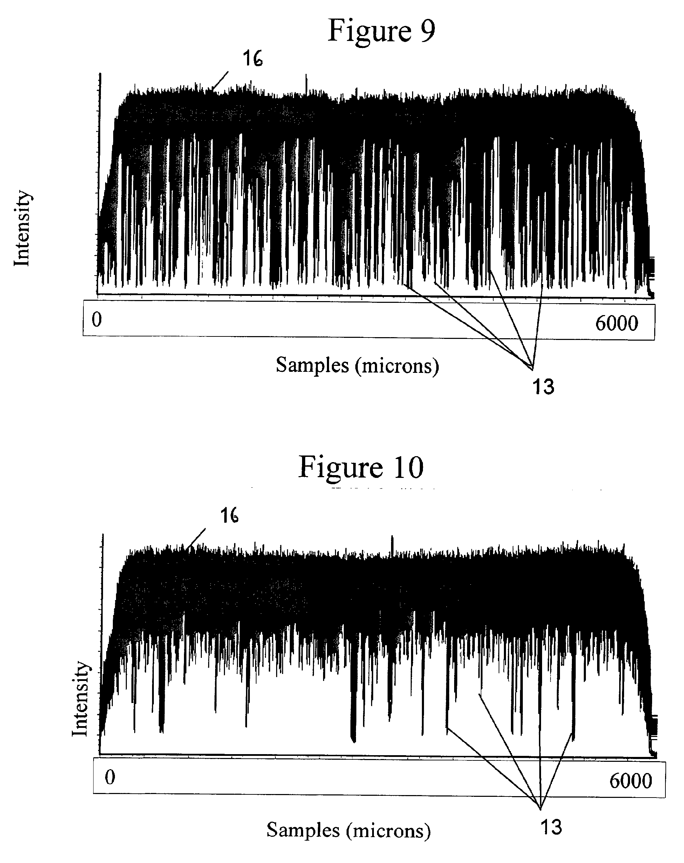

The experiment was repeated using 15nM FITC-MOPC antibody with similar results. Figure 9 shows the line amplitudes obtained from the streptavidin-coated beads and 15nM FITC-MOPC antibody solution. Figure 10 shows the line amplitudes obtained after substituting the streptavidin-coated beads with SAM beads.

## Claims

1. A method for performing a non-separation assay for determining the level of binding of one component to another, the method comprising the steps of:
providing a first component incorporating a fluorescent probe dissolved or suspended in solution;
providing a substantially opaque particle onto or into which is incorporated at least one of binding sites for the first component and a flourescent component;
immersing the opaque particle in a solution or suspension of the first component;
allowing the opaque particle to settle out of the solution, or be transported to a fixed position by an applied force;
illuminating the solution and opaque particle with a beam of light such that the opaque particle is in the foreground and attenuates the illuminating beam before it passes into the solution beyond;
determining the intensity of received light from the first component over an area of the sample with an imaging or scanning detector from the same side of the sample as the illuminating light; and
determining the position of the second component in the sample by detecting attenuation of the received light from the sample and/or the intensity of fluorescence from the second flourescent component.

2. A method according to claim 1, wherein the flourescent component incorporated in the opaque particle is a dye.

3. An apparatus for performing a non-separation assay, the apparatus comprising:
a opaque particle onto or into which is incorporated binding sites for a first component and/or a second flourescent component;
means for containing and presenting the first component and opaque particle in solution to an optical detector;
means for illuminating both the first component and the opaque particle with at least one illumination wavelength, the diameter of the beam or beams of light being no larger than twice the diameter of the opaque particle at the point of focus; and
means for measuring the intensity of the received light with at least one detection channel sensitive to the emission from the fluorophore incorporated in the first component.

4. An apparatus according to claim 3 further comprising at least one additional detection channel sensitive to the emission from the flourescent component incorporated in the opaque particle and insensitive to the fluorophore incorporated in the first component.

5. An apparatus according to claim 3 or 4, further comprising means for performing a signal processing algorithm to pick out and isolate the intensity value of the first component bound to the opaque particle amongst the continuous background signal from the unbound first component by:
firstly determining the position of the opaque particle through the detection of an attenuation in the signal from at least one detection channel sensitive to the emission from the fluorophore incorporated in the first component and/or through the detection of an increase in the signal from at least one detection channel sensitive to the emission from the fluorophore incorporated in the opaque particle; and
secondly, measuring the intensity value from the detection channel sensitive to the emission from the fluorophore incorporated in the first component.

6. An apparatus according to claim 3, 4 or 5, wherein the first component is one of a fluorescently labelled protein, ligand, enzyme, enzyme substrate, nucleic acid sequence, small molecule, viral particle, bacterium, or biologically-coated microbead.

7. An apparatus according to claim 3 to 6, wherein the opaque particle is one of a polymeric bead containing an opaque filler or an opaque inorganic bead, such beads being coated with, made of or incorporating a protein, receptor, ligand, enzyme, substrate, cell membrane, nucleic acid sequence or plastic.

8. An apparatus according to any of claims 3 to 7, wherein the opaque particle is magnetic, paramagnetic, electrically charged or is of higher density than the sample solution to facilitate settling out of the particle by application of a magnetic field, electrophoresis, static charge, gravity or centrifugation.

9. An opaque particle for use in the method of claims 1 and 2, comprising binding sites for a first component and/or a flourescent component.
